# NEW EUROPEAN PATENT SPECIFICATION

(11) **EP 1 115 380 B2**
(45) Date of publication and mention of the opposition decision: **12.03.2008**
(45) Mention of the grant of the patent: 26.05.2004
(21) Application number: 99949133.5
(22) Date of filing: 22.09.1999
(51) Int. Cl.: A61K 9/12, A61K 9/14, A61K 31/7036, A61K 38/12

(54) **MICRONISED PHARMACEUTICAL COMPOSITIONS**
MICRONISIERTE PHARMAZEUTISCHE ZUSAMMENSETZUNGEN
COMPOSITIONS PHARMACEUTIQUES MICROFINES

(30) Priority: 23.09.1998 GB 9820746
(43) Date of publication of application: 18.07.2001
(73) Proprietor: Pharmax Limited, Bexley, Kent DA5 1NX (GB)
(72) Inventor: FLYNN, Richard, Anthony, Wilmington,Kent DA2 7PQ (GB); GOLDMAN, Martin, Harris, London N20 0HT (GB); LOVELY, James, Richard, London SW5 9SG (GB)
(74) Representative: Green, Mark Charles
(86) International application number: PCT/GB1999/003172
(87) International publication number: WO 2000/016745

(56) References cited:
- EP-A- 0 388 621
- EP-A- 0 398 631
- WO-A-97/37708
- WO-A-98/14174
- WO-A-98/20836
- ROSE H. D. ET AL: "Evaluation of Sodium Colistimethate aerosol in gram negative Infections of the respiratory Tract" J. OF CLINICAL PHARMACOLOGY AND THE J. OF NEW DRUGS, vol. 10, no. 4, 1970, pages 274-281, XP000901745
- LITTLEWOOD J.M. ET AL: "Aerosol antibiotic treatment in cystic fibrosis" ARCHIVES OF DISEASE IN CHILDHOOD, vol. 68, 1993, pages 788-792, XP000901743
- P. J. Atkins et al., Drugs Pharm. Sci.1992, 54, 155 - 185
- D. Ganderton et al., Advances in Pharmaceutical Sciences, Academic Press , London, 1992 , 165 - 191
- M. P. Timsina et al., Int. J. Pharmaceutics, 1994, 101, 1 - 13
- P. M. O'Byrne, J. Aerosol Med., 1995, 8 Suppl. 3, 39 - 49, Abstract
- Römpps Chemie-Lexikon, 8. Auflage, Franck'sche Verlagshandlung 1983,Band 3, Seiten 1777 - 9, Eintrag "Hydration"
- D. Prime et al., Advanced Drug Delivery Reviews 1997, 26, 51 - 58
- J. M. Goldmann et al., Thorax 1990, 45, 939 - 940
- M. L. Everad et al., Thorax 1995, 50, 517 - 519
- B. J. Meakin et al., Drug Emission Efficacy of Turbospin, A New Single Dose Dry Powder Inhaler, 14th Pharmaceutical TEchnology Conference, Barcelona - Spain, April, 4th, 5th, and 6th, 1995
- H.G.Brittain (Ed.), Physical Characterization of Pharmaceutical Solids,Drugs and the Pharmaceutical Sciences Volume 70, 175 - 179, Marcel Dekker, Inc. (1995)
- F. M. Etzler et al., Part. Part. Syst. Charact.,1995, 12, 217 - 224

## Description

The present application relates to improvements in or relating to pharmaceutical compositions comprising micronised colistin sulphomethate sodium.

### Background and Prior Art

Colistin is an anti-bacterial cationic cyclic polypeptide belonging to the polymixin group. It is produced as a secondary metabolite of *Bacillus polymyxa* var. colistinus. Treatment of colistin base with formaldehyde and sodium bisulphite results in the production of colistin sulphomethate sodium. This is described in Japanese patent 4898/1957. The product is a crystalline powder which is soluble in water.

Colistin sulphomethate sodium is a combination of the negatively charged molecular ion colistin sulphomethate with positive sodium ions. It should be carefully distinguished from colistin sulphate. Both are described in the European Pharmacopoeia.

Colistin is of particular benefit in the treatment of serious infections caused by bacterial pathogens such as *Pseudomonas aeruginosa, Escherichia coli* and Klebsiella *sp.* An important property of colistin is that bacteria which are sensitive to the drug do not readily acquire resistance. Colistin as a pharmaceutical may be prepared into numerous different preparations, e.g. topical, bladder irrigation, oral such as tablets, or as intravenous or intra-muscular injections.

Colistin sulphate can be prepared from colistin. It is currently used to treat gram negative infections of the body such as intestinal infections due to various micro-organisms and, usually in association with other antibiotics, for the suppression of bowel flora. As noted above, colistin sulphate should be distinguished from colistin sulphomethate sodium.

Colistin sulphomethate sodium can also be prepared. It exists as a white to slightly yellow hygroscopic powder. It is commercially supplied at a particle size of 100-200 µm mass median diameter. The powder is highly soluble in water and as such is used for parenteral administration. As a powder, colistin sulphomethate sodium must be stored in air-tight containers, preferably protected from the light. Colistin sulphomethate sodium is used in the treatment of infections in patients suffering from cystic fibrosis, a genetic disease which affects many body systems, and which develops at a young age. Various glands of the body do not function properly. The disease is marked by a malfunction of the glands in the lining of the bronchial tubes. Instead of producing their normal thin mucus, the bronchial glands produce a thick, sticky mucus that stagnates in the tubes. Microbes are able to multiply readily, causing serious respiratory infections ultimately leading to respiratory failure. It is known that colistin sulphomethate sodium is effective in treatment of infections caused by these microbes e.g. *Pseudomonas aeruginosa.* The usual form of administration is as a solution for inhalation after nebulisation. The nebulised solution is prepared by taking a vial in which there is a known dosage of colistin sulphomethate sodium powder, injecting water into the vial and then inhaling the solution into the lungs through a nebuliser. Colistin sulphomethate sodium is poorly absorbed into the bloodstream. This is preferred as the bacteria can be attacked in the mucus which lines the lungs during illness.

Whilst jet nebulisation therapy has been shown to be successful, the nebulisation technique has several drawbacks. Jet nebulisers utilise compressed gases (usually air) to convert a drug solution into a spray. The compressed air passes through a narrow venturi orifice and negative pressure is created. Liquid is drawn from a fluid reservoir through a feed tube, fragments into droplets, and is accelerated to a velocity sufficient for more than 99% of the droplet mass to impact on baffles or on the nebuliser where droplets coalesce and drain back into the fluid reservoir. Only 1% of the aerosol mass leaves the nebuliser directly. The outgoing air becomes saturated with water derived from liquid retained in the nebuliser, and this has two important consequences: Firstly, the nebuliser is cooled and reaches an equilibrium temperature approximately 10°C below ambient, so that the patient inhales a relatively cold spray. Secondly, the evaporation of water causes the concentration of solutes to increase with time.

There are many different designs of nebuliser available which use different flow rates of compressed gas. The output from these nebulisers will all be different and accordingly it is difficult for a patient to ensure that a constant dose is administered. The nebulisers themselves are bulky due to the compressors which are required. Although described as being transportable, the nebuliser/compressor system is not truly portable. When they are undergoing treatment, patients need to remain connected to the mouthpiece of the nebuliser for approximately 20 minutes in order to complete the therapy and in order to ensure that the correct dose is administered. An electrical supply is needed to run the nebuliser.

It will be seen from the above that, although colistin sulphomethate sodium is a valuable pharmaceutical in the treatment of infections occurring during cystic fibrosis and other bacterial infections, there are a number of disadvantages which mean that it is not widely accepted as a treatment regime, particularly for infants. It has been determined that many of the problems arise from the preferred delivery method described above, i.e. as a nebulised liquid.

WO 95/00128 (Astra) describes the delivery to the lungs of dry powder polypeptides. An enhancer compound is used to promote absorption into the systemic circulation. In contrast, colistin sulphomethate sodium is used very locally in the lungs - absorption into the bloodstream is not an objective.

US-A-5,767,068 (Pathogenesis) describes the separation and use of individual components of colistin sulphate. Colistin sulphate is separated into individual components in free base form. Such components are described by Ebverdam, Larsen and Lund (Journal of Chromatography, 218 (1981) 653-661).

WO-A-98/20836 is to be noted as the international publication which is equivalent to US-A-5,767,068.

J. of Clinical Pharmacology and the J. of New drugs, 1970(10),274-281, describes sodium colistimethate aerosols for use in the treatment of gram-negative infections of the respiratory tract. The aerosol is prepared by dissolving sodium colistimethate sterile powder in sterile water. When administered through a suitable nebuliser the aerosol has a particle size of 1-7 microns.

Archives of Diseases in Childhood, 68,1993, 788-792, describes the treatment of cystic fibrosis using aerosols. The paper refers to the delivery of micronised gentamicin powder using a Rotahaler (registered trade mark) . It was found that the powder caused coughing. The paper concludes that aerosol forms of drugs delivered through a nebuliser are more suitable for treatment of cystic fibrosis.

### Summary of Invention

It has now been discovered that micronised colistin sulphomethate sodium in powder form can be administered to the airways of a patient using a powder dose inhalation device. The micronised colistin may be used alone or with a carrier, such as lactose.

The invention provides micronised particles of colistin sulphomethate sodium as claimed in claim 1.

According to the present invention, there is firstly provided the use of micronised colistin sulphomethate sodium in a method of treatment of the human body, particularly in the treatment of bacterial infections of the pulmonary system, most particularly in the treatment of secondary infections in patients suffering from cystic fibrosis, by powder inhalation.

According to a further aspect of the present application, there is provided a pharmaceutical composition comprising micronised colistin sulphomethate sodium and a powder carrier, in the absence of free liquid, in powder form.

According to a yet further aspect of the present invention, there is provided a pharmaceutical dosage form suitable for use with a dry powder inhaler comprising micronised colistin sulphomethate sodium, optionally together with a powder carrier, and a container. The container is preferably a capsule.

### Detailed Description

Micronised colistin sulphomethate sodium may be defined as being a powder wherein at least 90% by volume of the powder comprises particles have a diameter of less than 10 micrometers. Most preferably, at least 50% of the particles have a diameter of less than 8 micrometers. More preferably, at least 25% of the particles have a diameters of less than 6 micrometers.

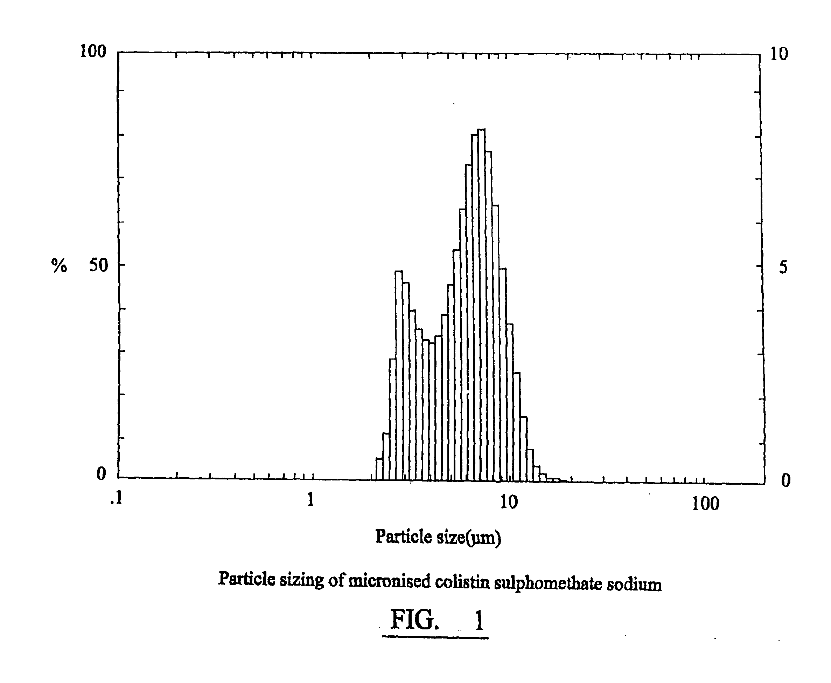

Figure 1 shows a particle size analysis of micronised colistin sulphomethate sodium.

Medicaments for administration by inhalation should be of a controlled particle size in order to achieve maximum penetration into the lungs, a suitable particle size range being 0.01-10, usually 1-8 micrometers. Particle sizes may be measured by a number of methods, e.g. by laser diffraction or microscope analysis.

Micronised colistin sulphomethate sodium may be prepared by fluid energy milling, ball milling, spray drying or precipitation. The colistin sulphomethate sodium may be administered in conjunction with a carrier. The carrier may be any non-toxic material which is chemically inert to the colistin sulphomethate sodium and will be acceptable for inhalation or for administration. Examples of carriers which may be used include inorganic salts, e.g. sodium chloride or calcium carbonate; organic salts, e.g. sodium tartrate or calcium lactate; organic compounds, e.g. urea; monosaccharides, e.g. lactose, arabinose or dextrose; disaccharides, e.g. maltose or sucrose; polysaccharides, e.g. starches and dextrans. A particularly preferred carrier is a lactose, e.g. crystalline lactose.

The present invention also provides a method for preparing a composition of the invention which comprises mixing together micronised colistin sulphomethate sodium and a carrier. The colistin sulphomethate sodium and the carrier may be blended in a drum, hoop or Y-cone blender as known in the art.

The carrier does not have to have the same particle size specification as the colistin sulphomethate sodium. The carrier may in fact generally be of a larger particle size than that of the colistin sulphomethate sodium in order to facilitate delivery from the inhalation device and yet not be deposited in the finer airways of the lungs. The inclusion of a carrier may ease dosage of pharmaceutical and carrier into capsules. Preferably at least 50%, and more desirably at least 70% by volume of the carrier particles have an effective particle size in the range of 30 to 150, especially 30 to 80, micrometers. The admixture of pharmaceutical and carrier may contain up to 75% by weight, more preferably up to 50% by weight of carrier. Generally the ratio of colistin sulphomethate sodium will be in the range of 5:1 to 1:2 preferably 4:1 to 1:1 by weight.

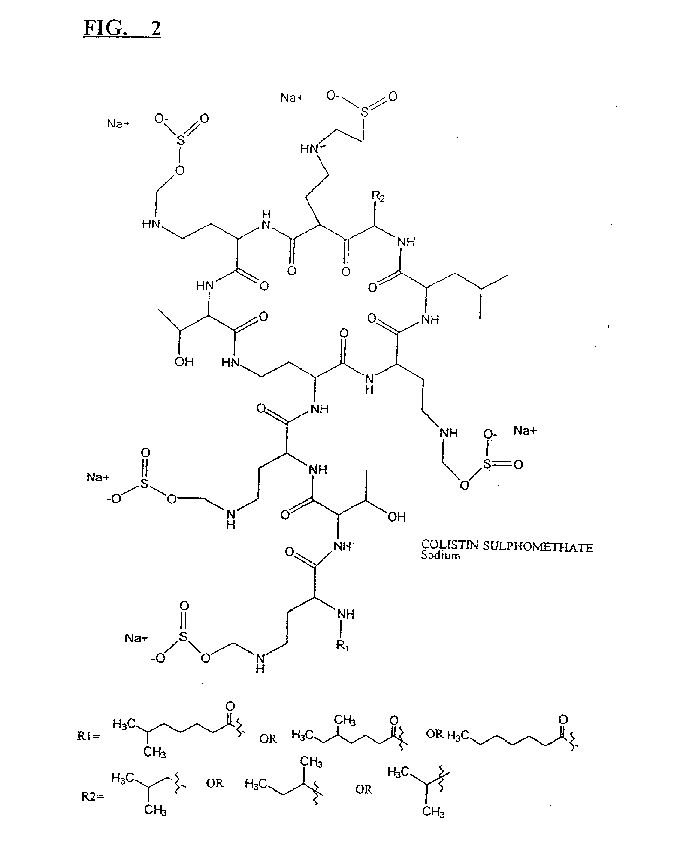

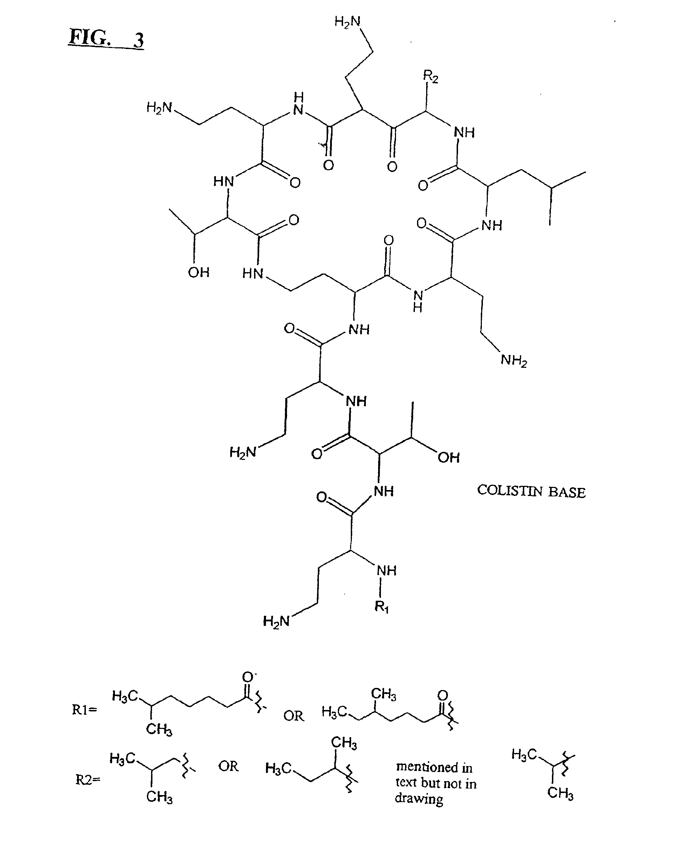

Colistin sulphomethate sodium is a negatively charged molecular ion with positively charged sodium counter ions. Figure 2 shows the structure. There are five sulphomethate groups (CH₂-OSO₂⁻). In contrast, Pathogenesis are producing a neutral base shown in Figure 3. US-A-5,767,068 refers to variable groups R₁ and R₂; R₁ is identified as 6-methyloctanoyl or 6-methylheptanoyl, and R₂ as sec-butyl, isobutyl or isopropyl.

It has now been surprisingly found that the negatively charged colistin sulphomethate ion (preferably in its sodium form) can be delivered to the lungs. As absorption into the blood stream is not wanted, the negatively charged ion is preferred to the base colistin.

It has been found that colistin sulphomethate sodium is a mixture of at least ten components. Tests carried out on mixtures of antibacterial preservatives show that the mixture of components found in colistin sulphomethate sodium show synergy of activity against gram negative microbial organisms.

It has been surprisingly found that water absorption of a micronised powder is comparatively low, e.g. approximately 5-7% by weight under normal atmospheric conditions. It has also been found that the micronised powder does not stick together. In powders having a larger particle size, the particles can stick together because of static forces. This sticking occurs with colistin and colistin sulphate. However, this is not found in the colistin sulphomethate sodium of the present invention. This is a further surprising advantage of the present application.

In addition to the micronised colistin sulphomethate sodium and, optionally, the carrier, the composition may contain other ingredients, such as colouring matter or flavouring agents such as saccharine, which may be present in inhalant compositions. Antistatic agents may also be added, e.g. as described in GB-A-2269992 (Rhone-Poulenc Rorer Ltd). It is preferred to use the minimum of such other ingredients.

The powder formulation may contain other pharmaceutical ingredients such as bronchodilators e.g. salbutamol Such other pharmaceutical ingredients preferably have an effective particle size similar to that of the colistin. The bronchodilatory drug will be delivered in very small (microgram) quantities. For example a capsule may contain from 50 to 15.0, e.g. 125, milligrams of colistin sulphomethate sodium and from 1 to 250, e.g. 200, micrograms of salbutamol.

The micronised powder may be delivered to the lungs through a specialised powder inhalation device. Most preferred is location of the powdered pharmaceutical within a.hard capsule or a blister package. The capsule or blister is ruptured or broached within the inhaler device, thereby enabling the powder to be inhaled through the mouthpiece as air is sucked in.

There is also provided, therefore, as a further feature of the invention, a dosage unit comprising a capsule containing colistin sulphomethate sodium, preferably in the form of a pharmaceutical composition of the present invention. The capsule may be formed of gelatin or a plastics material.

By carefully controlling the conditions under which capsules and blisters are filled, the final moisture level in the product can be kept to below 15 wt %, preferably below 5 wt %. The humidity level is preferably below 25% RH, most preferably below 15% RH. The low moisture level is important for product stability, and enables the product to be filled with minimal static effects. Flow out of the capsule or blister is also improved.

By careful selection of capsule and packaging components, stability and dosing can be controlled. The level of lubricant is kept low (preferably below 0.2% wt %). Capsules for oral use usually contain 2-3 wt % lubricant. Mould lubricant could interact with the dry powder. Capsule integrity is important, and accordingly a peelable lid to the blister package is preferred to a conventional "push out" seal. The blister may be, e.g. aluminium (40-50 µm thick) laminated with PVC (50-70 µm thick) and PA (20-30 µm thick). The peelable seal may be formed of soft aluminium (18-22 µm thick) laminated with PE7 (20-25 µm thick) .

The amount of composition contained in the capsule will, of course, depend upon the desired dosage. However, the capsule suitably contains from 10 to 200 milligrams, most preferably 30 to 150 milligrams of the colistin sulphomethate sodium. The colistin sulphomethate sodium may be delivered with or without a carrier. If a carrier is used then clearly a larger amount of the mix of carrier and pharmaceutical is required. It has been found that the capsule should contain a larger dose of drug than the amount which will actually be delivered to the lungs. Dosages are usually expressed in "units". 80 mg of colistin sulphomethate is equivalent to approximately 1 million units of colistin sulphomethate. One unit of colistin sulphomethate is contained in 0.00007874 mg of the first International Reference Preparation (1966) of colistin sulphomethate. Children with cystic fibrosis may be treated with nebulised colistin sulphomethate sodium at a level of 500,000 units, twice daily. The respirable fraction from a conventional nebuliser (CR 50 System 22) is approximately 9 mg of colistin sulphomethate sodium from a 500,000 unit dose. This can be tested using a multistage impinger and measuring mass collected at stages 3 and 4.

A preferred device for delivering the pharmaceutical composition according to the present invention is the Turbospin (Registered Trade Mark) originating from PH & T. This device uses a gelatin capsule which is pierced in the bottom by a single metal needle. When the patient inhales through the mouthpiece, air is drawn in through the tangentially arranged slits around the chamber. This spins the capsule and throws out the contents into the airstream. A flip top on the device allows up to three spare capsules to be stored. Another preferred device for delivering the pharmaceutical composition is the Aerohaler (Registered Trade Mark) from Boehringer Ingelheim. This device uses a hard gelatin capsule which is pierced by two metal needles in the side of a capsule. When the patient inhales through the mouthpiece, air enters the bottom of the chamber causing the capsule to spin and throw out its contents into the airstream. The unit holds six capsules in a carousel cartridge. When all six capsules have been used, the unit locks and it must be re-loaded. Other devices known in the art for delivery of encapsulated powders by inhalation can be used.

The capsule keeps the powder dry and thus in flowable form. The capsules should preferably be designed to protect their contents from light, e.g. they should be opaque or the capsules may be packed and/or stored in opaque containers, e.g. coloured or covered containers, or metal foil.

The invention is further described by reference to the following Examples, illustrated by the following figures.

Figure 1 shows a particle size analysis of micronised colistin sulphomethate sodium.

Figure 2 shows the structure of colistin sulphomethate with accompanying sodium ions.

Figure 3 shows a neutralised colistin base, as described in US-A-5,767,068.

### Examples

### Example 1

Micronised colistin sulphomethate sodium was produced by fluidised energy milling using a Hosokawa Alpine mill of powdered colistin sulphomethate sodium having an average particle size of approximately 100 µm supplied by Dumex Pharmaceuticals. A sample of the micronised colistin sulphomethate sodium was suspended in chloroform and the particle size analysed by a laser counter. Figure 1 shows the range of particle sizes of the micronised colistin.

### Example 2

Gelatin pharmaceutical capsules (standard size 2) were obtained from Shionogi Qualicaps. The capsules were filled using a standard dosator (Zanassi LZ64) under controlled temperature and humidity conditions (17°C/10%-15% RH). Colistin sulphomethate sodium was filled into the capsules either as pure micronised powder or together with a lactose carrier (lactose monohydrate lactochem pharmaceutical grade from Borculo Whey Products). The fills are as shown on Table 1.

**TABLE 1**

| **Run Number** | **Mix Used** | **Total Fill** |
|---|---|---|
| 1 | Colistin | 125 mg |
| 2 | Colistin/Lactose (1:1) | 165 mg |
| 3 | Colistin/Lactose (2:1) | 140 mg |
| 4 | Colistin/Lactose (4:1) | 130 mg |
| 5 | Colistin | 125 mg |

When colistin sulphomethate sodium is used alone, it flows well. Filling weights are standard. If a mixture of colistin to lactose as in Run 2 is used then the mixed powder flows well through the machine but there is sticking of the components of the dosator. Sticking reduces in Runs 3 and 4. Tests found respirable fractions in the region of 16 to 20 mg. This is the mass of colistin sulphomethate sodium collected on stages 3 and 4 of the multistage liquid impinger and equates to particles having a size less than about 3 to 4 micrometers.

### Example 3

Filled capsules produced from Runs 1 to 4 above were stored for nine months under various humidity conditions. There was no degradation or clumping of the colistin sulphomethate sodium. There was no noticeable clumping of colistin sulphomethate sodium on the capsule walls.

### Tests

Clinical trials were carried out. In one trial, the absorption of the powdered colistin sulphomethate sodium into the airways of the lungs was measured (specific airway conductance). It was found that 80% of patients, inhaling the micronised dry powder colistin sulphomethate sodium, were able to mobilise 80 mg of the drug, i.e. 1 mega unit. This is a very high uptake, and more than would be expected from a powdered drug. The powder does not cause irritation, and thus construction, of the lungs.

In a second trial, patients were given a premedication dose of 200 micrograms of salbutamol. This appeared to improve airway conductance.

In an alternative medication regime, the salbutamol can be mixed into the same capsule as the colistin sulphomethate sodium.

A further trial compared specific airway conductance, as measured by whole body plethysmography, of traditional nebulised colistin sulphomethate sodium and dry powder. There did not seem to be any noticeable difference.

## Claims

1. Micronised particles of colistin sulphomethate sodium in the absence of free liquid wherein at least 90% by volume of the micronised particles have a diameter of less than 10 micrometers, for use in the treatment of a pulmonary infection by powder inhalation, wherein the colistin sulphomethate sodium is not separated into component form and is present as a powder.

2. Micronised particles of colistin sulphomethate sodium as claimed in Claim 1 wherein the micronised powder is mixed with a powder carrier.

3. Micronised particles of colistin sulphomethate sodium as claimed in Claim 2 wherein the carrier is lactose.

4. A composition comprising micronised particles of colistin sulphomethate sodium as defined in Claim 1 and a powder carrier, in the absence of free liquid.

5. A composition as claimed in Claim 4 wherein the carrier is lactose.

6. A composition as claimed in Claim 4 or Claim 5 wherein the ratio of colistin sulphomethate sodium to carrier is from 5:1 to 1:2 by weight.

7. A composition as claimed in Claim 4 or Claim 5 wherein the ratio of colistin sulphomethate sodium to carrier is from 4:1 to 1:1 by weight.

8. The composition a claimed in any one of Claims 4 to 7 wherein at least 50% by volume of the carrier particles have an effective particle size in the range of 30-150 micrometers.

9. A composition as claimed in any one of Claims 4 to 8 wherein at least 50% by volume of the micronised colistin sulphomethate sodium particles have a particle diameter of less than 8 micrometers.

10. A composition as claimed in any one of Claims 4 to 9 wherein at least 25% of the particles of micronised colistin sulphomethate sodium have a diameter of less than 6 micrometers.

11. A composition as claimed in any one of Claims 4 to 10 wherein the micronised colistin sulphomethate sodium is prepared in the desired particle size range using a fluid energy mill.

12. A process for the preparation of a composition as claimed in any one of Claims 4 to 11 which comprises mixing micronised colistin sulphomethate sodium and a powder carrier.

13. A pharmaceutical dosage form suitable for use with a dry powder inhaler comprising micronised colistin sulphomethate sodium as claimed in Claim 1 or a composition according to any one of Claims 4 to 11 and a container, said dosage having a content of below 10 wt % water of hydration but having an absence of free liquid.

14. A pharmaceutical dosage form according to Claim 13 wherein the container is a hard gelatin capsule.

15. A capsule containing micronised colistin sulphomethate sodium as claimed in Claim 1.

16. A capsule as claimed in Claim 15 containing from 10 to 200 micrograms of micronised colistin sulphomethate sodium.

17. A capsule as claimed in Claim 15 containing from 30 to 150 milligrams of micronised colistin sulphomethate sodium.

18. A capsule as claimed in any one of Claims 15 to 17 further comprising a carrier.

19. A capsule as claimed in Claim 18 when the carrier is lactose.

20. A capsule according to any one of Claims 15 to 19 which is opaque.

21. A capsule according to any one of Claims 15 to 19 or a composition according to any one of Claims 4 to 11 packed in an opaque container.

22. A capsule containing micronised colistin sulphomethate sodium as claimed in Claim I in unit dosage form.

23. A capsule according to any one of Claims 15 to 22 which additionally comprises a micronised bronchodilatory drug.

24. A capsule according to Claim 23 wherein the bronchodilatory drug is salbutamol.

25. A capsule according to Claim 23 or Claim 24 which comprises from 5 to 150 milligrams of colistin sulphomethate sodium and from 1 to 250 micrograms of bronchodilatory drug.

## Patentansprüche

1. Mikronisierte Teilchen von Colistinsulfomethat-Natrium in Abwesenheit freier Flüssigkeit, wobei mindestens 90 Vol.-% der mikronisierten Teilchen einen Durchmesser von weniger als 10 Mikrometern aufweisen, für die Verwendung bei der Behandlung einer Lungeninfektion durch Pulverinhalierung, wobei das Colistinsulfomethat-Natrium nicht in Komponentenform aufgeteilt wird und als Pulver vorliegt.

2. Mikronisierte Teilchen von Colistinsulfomethat-Natrium nach Anspruch 1, wobei das mikronisierte Pulver mit einem Pulverträger gemischt wird.

3. Mikronisierte Teilchen von Colistinsulfomethat-Natrium nach Anspruch 2, wobei es sich bei dem Träger um Lactose handelt.

4. Zusammensetzung, die mikronisierte Teilchen von Colistinsulfomethat-Natrium wie in Anspruch 1 definiert und einen Pulverträger in Abwesenheit freier Flüssigkeit umfasst.

5. Zusammensetzung nach Anspruch 4, wobei es sich bei dem Träger um Lactose handelt.

6. Zusammensetzung nach Anspruch 4 oder Anspruch 5, wobei das Verhältnis von Colistinsulfomethat-Natrium zum Träger 5:1 bis 1:2, auf das Gewicht bezogen, beträgt.

7. Zusammensetzung nach Anspruch 4 oder Anspruch 5, wobei das Verhältnis von Colistinsulfomethat-Natrium zum Träger 4:1 bis 1:1, auf das Gewicht bezogen, beträgt.

8. Zusammensetzung nach einem der Ansprüche 4 bis 7, wobei mindestens 50 Vol.-% der Trägerteilchen eine wirksame Teilchengröße im Bereich von 30 - 150 Mikron aufweisen.

9. Zusammensetzung nach einem der Ansprüche 4 bis 8, wobei mindestes 50 Vol-% der mikronisierten Colistinsulfomethat-Natrium-Teilchen einen Teilchendurchmesser von weniger als 8 Mikrometer aufweisen.

10. Zusammensetzung nach einem der Ansprüche 4 bis 9, wobei mindestens 25 Vol-% der mikronisierten Colistinsulfomethat-Natrium-Teilchen einen Durchmesser von weniger als 6 Mikrometer aufweisen.

11. Zusammensetzung nach einem der Ansprüche 4 bis 10, wobei die mikronisierten Colistinsulfomethat-Natrium-Teilchen im erwünschten Teilchendurchmesserbereich mit Hilfe einer Fluidenergiemühle zubereitet werden.

12. Verfahren für die Zubereitung einer Zusammensetzung nach einem der Ansprüche 4 bis 11, das das Mischen von

13. Pharmazeutische Dosierform, die für die Verwendung mit einem Trockenpulverinhalator geeignet ist, der mikronisiertes Colistinsulfomethat-Natrium nach Anspruch 1 oder eine Zusammensetzung nach einem der Ansprüche 4 bis 11 und einen Behälter umfasst, wobei die Dosis einen Gehalt von weniger als 10 Gew.-% Hydratationswasser jedoch keine freie Flüssigkeit aufweist.

14. Pharmazeutische Dosierform nach Anspruch 13, wobei der Behälter aus einer harten Gelatinekapsel besteht.

15. Kapsel, die mikronisiertes Colistinsulfomethat-Natrium nach Anspruch 1 enthält.

16. Kapsel nach Anspruch 15, die 10 bis 200 Mikrogramm mikronisiertes Colistinsulfomethat-Natrium enthält.

17. Kapsel nach Anspruch 15, die 30 bis 150 Milligramm mikronisiertes Colistinsulfomethat-Natrium enthält.

18. Kapsel nach einem der Ansprüche 15 bis 17, die des Weiteren einen Träger umfasst.

19. Kapsel nach Anspruch 18, wenn es sich bei dem Träger um Lactose handelt.

20. Kapsel nach einem der Ansprüche 15 bis 19, die opak ist.

21. Kapsel nach einem der Ansprüche 15 bis 19 oder Zusammensetzung nach einem der Ansprüche 4 bis 11, die in einem opaken Behälter verpackt ist.

22. Kapsel, die mikronisiertes Colistinsulfomethat-Natrium nach Anspruch 1 in einer Einheitsdosenform enthält.

23. Kapsel nach einem der Ansprüche 15 bis 22, die des Weiteren ein mikronisiertes Bronchodilatator-Medikament umfasst.

24. Kapsel nach Anspruch 23, wobei es sich bei dem Bronchodilatator-Medikament um Salbutamol handelt.

25. Kapsel nach Anspruch 23 oder Anspruch 24, die 5 bis 150 Milligramm Colistinsulfomethat-Natrium und 1 bis 250 Mikrogramm Bronchodilatator-Medikament umfasst.

## Revendications

1. Particules micronisées de sulfométhate de colistine sodique, en l'absence de liquide libre, dans lesquelles au moins 90% en volume des particules micronisées ont un diamètre de moins de 10 micromètres en vue d'une utilisation visant le traitement d'une infection pulmonaire par inhalation de poudre, dans lesquelles le sulfométhate de colistine sodique n'est pas séparé sous la forme de composants et est présent en tant que poudre.

2. Particules micronisées de sulfométhate de colistine sodique selon la revendication 1, dans lesquelles la poudre micronisée est mélangée à un véhicule pulvérisé.

3. Particules micronisées de sulfométhate de colistine sodique selon la revendication 2, dans lesquelles le véhicule est le lactose.

4. Composition comprenant des particules micronisées de sulfométhate de colistine sodique selon la revendication 1 et un véhicule pulvérisé, en l'absence de liquide libre.

5. Composition selon la revendication 4, dans laquelle le véhicule est le lactose.

6. Composition selon la revendication 4 ou la revendication 5, dans laquelle le rapport du sulfométhate de colistine sodique au véhicule va de 5:1 à 1:2 en poids.

7. Composition selon la revendication 4 ou la revendication 5, dans laquelle le rapport du sulfométhate de colistine sodique au véhicule va de 4:1 à 1:1 en poids.

8. Composition selon l'une quelconque des revendications 4 à 7, dans laquelle au moins 50% en volume des particules du véhicule ont une taille effective de particule dans le domaine de 30-150 micromètres.

9. Composition selon l'une quelconque des revendications 4 à 8, dans laquelle au moins 50% en volume des particules micronisées de sulfométhate de colistine sodique ont un diamètre de particule de moins de 8 micromètres.

10. Composition selon l'une quelconque des revendications 4 à 9, dans laquelle au moins 25% en volume des particules micronisées de sulfométhate de colistine sodique ont un diamètre de moins de 6 micromètres.

11. Composition selon l'une quelconque des revendications 4 à 10, dans laquelle le sulfométhate de colistine sodique micronisé est préparé dans le domaine de particules désiré par utilisation d'un désintégrateur à jet d'air.

12. Procédé en vue de la préparation d'une composition selon l'une quelconque des revendications 4 à 11, qui comprend le mélange du sulfométhate de colistine sodique micronisé et d'un véhicule pulvérisé.

13. Forme de dosage pharmaceutique appropriée à une utilisation avec un inhalateur à poudre sèche, comprenant le sulphométhate de colistine sodique micronisée selon la revendication 1, ou une composition selon l'une quelconque des revendications 4 à 11 et un récipient, ledit dosage ayant une teneur de moins de 10% en poids d'eau d'hydratation, mais ayant une absence de liquide libre.

14. Forme de dosage pharmaceutique selon la revendication 13, dans laquelle le récipient est une capsule de gélatine dure.

15. Capsule contenant du sulfométhate de colistine sodique micronisé selon la revendication 1.

16. Capsule selon la revendication 15, contenant de 10 à 200 microgrammes de sulfométhate de colistine sodique micronisée.

17. Capsule selon la revendication 15, contenant de 30 à 150 milligrammes de sulfométhate de colistine sodique micronisée.

18. Capsule selon l' une quelconque des revendications 15 à 17, comprenant en outre un véhicule.

19. Capsule selon la revendication 18, lorsque le véhicule est le lactose.

20. Capsule selon l'une quelconque des revendications 15 à 19, qui est opaque.

21. Capsule selon l'une quelconque des revendications 15 à 19 ou composition selon l'une quelconque des revendications 4 à 11, emballé(e) dans un récipient opaque.

22. Capsule contenant le sulfométhate de colistine sodique micronisé selon la revendication 1 dans une forme de dosage unitaire.

23. Capsule selon l'une quelconque des revendications 15 à 22, qui comprend en outre un médicament bronchodilatateur micronisé.

24. Capsule selon la revendication 23, dans laquelle le médicament bronchodilateur est le salbutamol.

25. Capsule selon la revendication 23 ou la revendication 24, qui comprend de 5 à 150 milligrammes de sulfométhate de colistine sodique et de 1 à 250 milligrammes de médicament bronchodilateur.
